(19) Europäisches Patentamt
European Patent Office
Office européen des brevets

(11) **EP 1 595 944 A1**

(12) **EUROPEAN PATENT APPLICATION**
published in accordance with Art. 158(3) EPC

(43) Date of publication:
16.11.2005 Bulletin 2005/46

(51) Int Cl.[7]: **C12N 5/00**, C08L 101/14

(21) Application number: 04712684.2

(22) Date of filing: 19.02.2004

(86) International application number:
PCT/JP2004/001856

(87) International publication number:
WO 2004/074463 (02.09.2004 Gazette 2004/36)

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 19.02.2003 JP 2003041391

(71) Applicant: WASEDA UNIVERSITY
Shinjuku-ku, Tokyo 169-8050 (JP)

(72) Inventors:
• MORI, Yuichi
Yokohama-shi, Kanagawa 236-0045 (JP)

• YOSHIOKA, Hiroshi
Kanagawa 257-0026 (JP)
• SATO, Yuko
Urayasu-shi Chiba 279-0031 (JP)
• YOSHIDA, Satoru
Higashiyamato-shi Tokyo 207-0021 (JP)
• OHTSUBO, Shinya
Chofu-shi Tokyo 182-0004 (JP)

(74) Representative: Wössner, Gottfried
HOEGER, STELLRECHT & PARTNER
Patentanwälte
Uhlandstrasse 14c
70182 Stuttgart (DE)

(54) **HYDROGEL FOR CELL SEPARATION AND METHOD OF SEPARATING CELLS**

(57) Cell/organism is separated by using a hydrogel capable of selectively moving a cell in accordance with a difference in the concentration of a physiologically active substance. The invention provides a hydrogel capable of conducting separation (fractionation, differential separation, fractional collection) of cell/organism having a variety of taxes, which could not be attained in the prior art.

Fig.1

**Description**

TECHNICAL FIELD

The present invention relates to a hydrogel for separating cell, microorganism and others, which is for separating cell and/or organism (hereinafter referred to as "cell/organism") by utilizing the chemotaxis or the property of moving according to the intensity of the property of a field (electrotaxis, magnetotaxis, phototaxis, thermotaxis, viscotaxis, etc.), and to a method of utilizing the hydrogel for separating cell/organism.

More precisely, the invention relates to a hydrogel for selectively moving a cell, microorganism and others by utilizing the property of moving according to the concentration of a physiologically active substance that is intrinsic to many organisms (chemotaxis), or utilizing the property of moving according to the intensity of the property of the field around them, to thereby separate the cell/organism (that is, for carrying out the operation of selective movement such as separation (fractionation, differential separation, fractional collection)), and to a method of utilizing the hydrogel for separating cell/organism.

Utilizing the hydrogel of the invention enables, for example, separation (fractionation, differential separation, fractional collection) of cell based on the difference in the chemotaxis to the factor associated with immune disorders of such as atopic dermatitis, allergy, rheumatism.

In addition, utilizing the hydrogel of the invention enables, for example, separation (fractionation, differential separation, fractional collection) of cell based on the difference in the chemotaxis to the factor associated with cancer cell metastasis relating to cancer therapy.

Further, utilizing the hydrogel of the invention enables, for example, separation (fractionation, differential separation, fractional collection) of cell based on the difference in the chemotaxis to the factor associated with induction, generation and regeneration of tissues and organs such as blood vessels and nerves relating to the field of regenerative medicine.

Still further, utilizing the hydrogel of the invention enables, for example, separation (fractionation, differential separation, fractional collection) of cell based on the difference in the motility associated with a screening technique for good sperms.

Still further, utilizing the hydrogel of the invention enables, for example, separation (fractionation, differential separation, fractional collection) of cell based on the difference in the taxis to an electric field (e.g., electrophoretic force).

BACKGROUND ART

When an organism (or its part) having motility reacts to external stimulation applied thereto and when its motility is recognized to be directional, then the property of this organism is referred to as taxis, and it is well known in the art. When the stimulation is caused by a substance, then the property is referred to as chemotaxis. On the other hand, when the stimulation is physical stimulation, then the property is referred to as electrotaxis, magnetotaxis, phototaxis, thermotaxis or viscotaxis depending on the type of the physical stimulation.

The organism having the mobility as above includes lower animals, plants, microorganisms, cells and organisms, and it is considered that almost all organisms on the earth will have taxis. With the recent development of studies relating to various taxes, it has become clarified that the taxes of organisms especially the taxes of cell participate in important biological functions.

For example, it has been clarified that blood vessel induction and regeneration requires growth of blood vessel endothelial cell, and blood vessels are induced and regenerated in accordance with the field having a concentration gradient of a blood vessel endothelial cell growth factor, or that is, they are induced and regenerated in accordance with the chemotaxis of blood vessel endothelial cell. In addition, it is well known that cancer cell having high auxotrophy secrete a blood vessel endothelial cell growth factor therefore inducing blood vessels into a cancer tissue from host blood vessels. Further, it has also been clarified that blood vessel systems are induced and regenerated by the gradation of the oxygen concentration around them. Since the important function of blood vessel systems is to supply oxygen to tissues and organs, it may be considered that the above is for inducing and regenerating blood vessel systems in a low-oxygen region to control the oxygen concentration therein. Accordingly, blood vessel endothelial cell have negative chemotaxis to oxygen.

Similarly, induction and regeneration of neurosystem are skillfully carried out by imparting a concentration gradient of a neurocyte growth factor to the field in living bodies. It is well known that immunity-related cell such as leucocytes react to an allergen that induces allergic reaction to exhibit the chemotaxis and leucocytes are accumulated in the reacted site. Further, there is increasing a possibility that cancer metastasis may be caused by cancer cell having high chemotaxis.

On the other hand, in development of novel anticancer agents, drugs having the property of inducing chemotaxis to immunity-related cell and also accumulating selectively in cancer tissue are specifically noted. Accordingly, development of drugs based on the chemotaxis is expected.

Apart from the above-mentioned chemotaxis, taxes by physical stimulation are also well known. For example, the growth of plants in the direction of light is well known as phototaxis. Further, cell having various electrotaxes in cell electrophoresis are known. Still further, it has become clarified that cell having high mobility such as sperms have a high correlation between the mobility and function.

As mentioned above, the taxes of organisms especially cells, in particular the chemotaxis thereof are extremely important property for function control of living bodies, and have become greatly noticed these days from the viewpoint of understanding biological phenomena, especially from the viewpoint of developing novel therapy for various immune disorders, cancers and others as mentioned above, and further from the viewpoint of developing regenerative medicine for inducing and regenerating diseased or damaged tissues or organs.

Studies and developments have heretofore been made relating to devices for measuring the taxes that are important functions of organisms. For example, there are, known a Boyden chamber system in which a porous membrane is disposed between a cell suspension and a chemotactic factor solution and the number of cell having moved to the factor side through the pores of the membrane is counted; and a method of using an array with a large number of fine paths formed in a silicon single-crystal substrate through which cell could barely pass, in place of a porous membrane, contacting a cell suspension with a chemotactic factor solution, and microscopically counting the number of cell having passed through the fine paths. However, when the apparatus or the method heretofore developed in the art is used, then the number of only cell having chemotaxis on a level not lower than a predetermined threshold value to a specific chemotactic factor could be only measured.

The tissues and organs that constitute our living bodies are formed of an extremely large variety of cell. Further, it is considered that the cell will continuously change in accordance with the environment around them. For example, the group of immunity-associated cell is composed of a variety of cell, and is classified into lymphocytes and macrophages. The lymphocytes include B lymphocytes and T lymphocytes; and the B lymphocytes are differentiated into many plasmocytes through external stimulation applied thereto. On the other hand, T lymphocytes are grouped into killer T cells, helper T cells, suppresser T cells and various T cell subpopulations, depending on the functions. It is being clarified that macrophages also have a variety of subpopulations.

Further, there exist a variety of cancer cell in cancer tissues, and it has been clarified that cancer cells have various distributions in point of the drug resistance, radiation resistance, proliferation capability and metastasis capability. In addition, it is well known that the stem cells existing in bone marrow are differentiated into various cells depending on the environment around them.

Naturally, a variety of such cell groups have the own taxes peculiar to them, and if it becomes possible to fractionate the cells due to the respective taxis thereof and if it becomes further possible to differentially separate and fractionally collect the cell having the respective taxis, then it may be a great advance for the clarification of cell functions. In addition, it may be considered that the above will make it possible to develop a novel therapeutic method for immunity-associated disorders and cancers for which any effective therapy is unknown at present, and to develop a technique of more effective regenerative medicine for tissues and organs.

However, as so mentioned hereinabove, according to the current technology of cell taxis measurement, it is in fact impossible to attain separation (fractionation, differential separation, fractional collection) of cell/organism having a variety of taxes.

DISCLOSURE OF THE INVENTION

An object of the invention is to provide a hydrogel for separating cell/organism and a method of separating cell/organism capable of solving the above-mentioned drawbacks in the prior art.

Another object of the invention is to provide a hydrogel capable of conducting the separation (fractionation, differential separation, fractional collection) of cell/organism having a variety of taxes, which cannot be attained in the prior art.

A further object of the invention is to provide a method of using the hydrogel for separating cell/organism in accordance with the taxis.

As a result of earnest study, the present inventors have found that separation of cell/organism by the use of a hydrogel having a specific constitution or a hydrogel capable of realizing a concentration difference in the inside of the gel, or between the inside and outside of the gel relating to a specific substance is extremely effective for attaining the above-mentioned object.

The hydrogel according to the present invention is based on the above discovery, and is a hydrogel for separating cell/organism, which is capable of selectively moving a cell/organism in accordance with a difference in the concentration of a physiologically active substance.

The present invention also provides, e.g., a hydrogel for separating cell/organism as described above, wherein the hydrogel shows a thermo-reversible sol-gel transition such that it assumes a sol state at a lower temperature and assumes a gel state at a higher temperature, and the gel is substantially insoluble in water at a temperature higher than the sol-gel transition temperature thereof.

The present invention further provides a method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,

contacting an aqueous solution containing a physiologically active substance with one face of the gel-forming composition in a gel state, and contacting a suspension of cell/organism with the other face of the composition in a gel state, at a temperature higher than the sol-gel transition temperature of the gel-forming composition,

transferring the cell/organism from the suspension into the composition in a gel state due to the chemotaxis induced by a concentration gradient, while providing the concentration gradient of the physiologically active substance in the inside of the composition in a gel state,

separating at least a portion of the composition in a gel state into which the cell/organism has been transferred, from the other portion of the composition, and

cooling the separated portion of the composition in a gel state to a temperature lower than the sol-gel transition temperature of the composition so as to convert the composition into a sol state, to thereby collect the cell/organism from the composition in a sol state.

The present invention further provides a method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,

adding a cell/organism to the gel-forming composition in a sol state at a temperature lower than the sol-gel transition temperature of the composition, to thereby suspend the cell/organism in the composition;

converting the sol-state composition into a gel state at a temperature higher than the sol-gel transition temperature, to thereby provide a composition in a gel state containing the cell/organism substantially uniformly dispersed therein,

contacting the composition in a gel state with an aqueous solution containing a physiologically active substance, at a temperature higher than the sol-gel transition temperature,

re-arranging the cell/organism in the composition in a gel state due to a difference in chemotaxis in accordance with a concentration gradient of the physiologically active substance, while transferring the physiologically active substance into the composition in a gel state so as to provide the concentration gradient of the physiologically active substance in the composition,

separating at least a portion of the composition in a gel state containing the cell/organism which has been re-arranged therein, from the other portion of the composition, and

cooling the separated portion of the composition in a gel state to a temperature lower than the sol-gel transition temperature of the composition so as to convert the composition into a sol state, to thereby collect the cell/organism from the composition in a sol state.

The present invention further provides a method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,

cooling the gel-forming composition to a temperature lower than the sol-gel transition temperature, to thereby convert the composition into a sol state, and substantially uniformly mixing a physiologically active substance in the composition in a sol state,

converting the sol-state composition into a gel state having a predetermined shape, at a temperature higher than the sol-gel transition temperature,

contacting the composition in a gel state with a suspension of cell/organism at a temperature higher than the sol-gel transition temperature, to thereby transfer the cell/organism from the suspension into the composition in a gel state,

The present invention further provides a method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,

positioning the gel-forming composition in a gel state in a field in which a physical property is continuously changed, to thereby provide a gradient of the physical property in the inside of the hydrogel,

contacting the gel-state composition with a suspension of cell/organism, and transferring the cell/organism in the suspension into the gel-state composition due to taxis induced by the gradient of the field property,

separating at least a portion of the gel-state composition containing the cell/organism having transferred therein, from the other portion of the composition, and

cooling the separated portion of the gel-state composition to a temperature lower than the sol-gel transition temperature of the composition so as to convert the composition into a sol state, to thereby collecting the cell/organism from sol-state the composition.

The present invention further provides a method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,

converting the gel-forming composition into a sol state at a temperature lower than the sol-gel transition temperature, and adding cell/organism to the sol, to thereby form a composition containing the cell/organism suspended therein,

converting the composition containing the cell/organism suspended therein, into a gel state at a temperature higher than the sol-gel transition temperature, to thereby form a composition in a gel state in which the cell/organism is substantially uniformly dispersed,

positioning the gel-forming composition in a gel state in a field in which a physical property is continuously changed, to thereby provide a gradient of the physical property in the inside of the gel,

re-arranging the cell/organism in the gel-state composition, which has been substantially uniformly distributed therein, in accordance with the gradient of the physical property and with a difference of the taxis of the cell/organism to the physical property,

separating at least a portion of the gel-state composition containing the cell/organism re-arranged therein, from the other portion of the composition, and

cooling the separated portion of the gel-state composition to a temperature lower than the sol-gel transition temperature of the composition so as to convert the composition into a sol state, to thereby collect the cell/organism from sol-state the composition.

In the invention having the above-mentioned constitution, it is possible to separate (fractionate, differentially separate, fractionally collect) cell/organism in accordance with the taxes by the use of a hydrogel (e.g., a hydrogel exhibiting a sol-gel transition of such that it is in a sol state at a low temperature but is gelled at a high temperature, the sol-gel transition is thermo-reversible, and its gel is substantially insoluble in water at a temperature higher than the sol-gel transition temperature). Specifically, in the invention, for example, it is possible to separate (fractionate, differentially separate, fractionally collect) cell/organism in accordance with the difference in the chemotaxis by producing a concentration gradient of a physiological substance (chemotactic factor) that induces the chemotaxis of the cell/organism in the hydrogel or by producing a difference in the concentration of the physiological substance between the inside and outside of the hydrogel.

Further, in the invention, it is possible to separate (fractionate, differentially separate, fractionally collect) cell/organism in accordance with the difference in the taxes to various properties of electric field, magnetic field, light intensity, temperature and viscosity, by producing the gradation of such property in the hydrogel for the cell/organism.

In one preferred embodiment of the invention, for example, using the hydrogel, an aqueous solution containing a physiologically active substance may be kept separated from a suspension of cell/organism for fractionation, at a temperature higher than the sol-gel transition temperature of the hydrogel, a concentration gradient of the physiologically active substance may be produced inside the hydrogel, and the cell/organism can be thereby transferred into the hydrogel from the cell/organism suspension based on the chemotaxis induced by the concentration gradient. As a result, since the motility or the moving distance of various cell/organism into the hydrogel differs depending on the difference in the chemotaxis of the cell/organism for fractionation, the hydrogel into which the cell/organism has moved or a part of the hydrogel that contains the cell/organism having a different taxis, or that is, having a different moving distance may be cut out, and cooled to a temperature lower than the sol-gel transition temperature of the hydrogel, and a sol state containing the cell/organism can be thereby produced. Next, a large quantity of a culture or a preservation medium for the cell/organism may be added to the sol to thereby dilute the polymer solution in order that the sol does not gel even at a temperature higher than the sol-gel transition temperature of the hydrogel, and thereafter the cell/organism may be separated (fractionated, differentially separated, fractionally collected) according to an ordinary fractionation method of centrifugation or membrane separation.

In another preferred embodiment of the invention, the hydrogel may be cooled to a temperature lower than the sol-gel transition temperature thereof to thereby make it in a sol state, and then cell/organism for fractionation may be added to it to prepare a suspension of the cell/organism. Next, the cell/organism suspension may be gelled by heating it up to a temperature higher than the sol-gel transition temperature of the hydrogel, to thereby produce a hydrogel with the cell/organism for fractionation substantially uniformly dispersed inside it. Next, the resulting hydrogel may be contacted with an aqueous solution containing a physiologically active substance (chemotactic factor) so as to move the physiologically active substance into the hydrogel, thereby producing a concentration gradient of the physiologically active substance in the hydrogel. With that, the cell/organism which have been substantially uniformly distributed inside the hydrogel may be then re-arranged in different sites in the hydrogel according to the concentration gradient, that is, according to the difference in the chemotaxis. Next, the respective sites of the hydrogel may be cut out, and the cell/organism may be separated (fractionated, differentially separated, fractionally collected) in accordance with the difference in the chemotaxis in the same manner as above.

Still another preferred embodiment of the invention comprises a step of cooling the hydrogel to a temperature lower than the sol-gel transition temperature thereof to thereby produce an aqueous solution of the sol state thereof, and then substantially uniformly mixing a physiologically active substance in the aqueous solution of the sol state; a step of heating the mixture of the sol state to a temperature higher than the sol-gel transition temperature thereof to produce a hydrogel having a predetermined shape; a step of contacting the predetermined shape-having hydrogel with a suspension of cell/organism at a temperature higher than the sol-gel transition temperature of the hydrogel; a step of collecting the predetermined shape-having hydrogel into which the cell/organism has moved, from the cell/organism suspension; and a step of cooling the thus-collected, predetermined shape-having hydrogel to a temperature lower than the sol-gel transition temperature thereof to thereby collect the fractionated and separated cell/organism as an aqueous solution of the sol state thereof. In this embodiment, the targeted cell/organism may be separated (fractionated, differentially separated, fractionally collected).

In still another preferred embodiment of the invention, the hydrogel may be put in a field in which the physical property thereof selected from the electric field intensity, the magnetic field intensity, the light intensity, the temperature and the viscosity thereof continuously varies to thereby produce a gradation of the property in the inside of the hydrogel, and then the hydrogel may be contacted with a suspension of cell/organism for fractionation to thereby move the cell/organism into the hydrogel from the cell/organism suspension according to the taxis induced by the gradation of the respective property. Depending on the difference in the taxis of the cell/organism for fractionation to the respective physical property, the motility or the moving distance of various cell/organism into the hydrogel varies. Next, the hydrogel into which the cell/organism has moved or a part of the hydrogel that contains the cell/organism having a different taxis, or that is, having a different moving distance may be cut out, and cooled to a temperature lower than the sol-gel transition temperature of the hydrogel, and a sol state containing the cell/organism may be thereby produced. Next, a large quantity of a culture or a preservative medium for the cell/organism may be added to the sol to thereby dilute the polymer solution in order that the sol does not gel even at a temperature higher than the sol-gel transition temperature of the hydrogel, and thereafter the cell/organism may be separated (fractionated, differentially separated, fractionally collected) according to an ordinary fractionation method of centrifugation or membrane separation.

In still another preferred embodiment of the invention, the hydrogel may be cooled to a temperature lower than the sol-gel transition temperature thereof to thereby make it in a sol state, and then cell/organism for fractionation may be added to it to prepare a suspension of the cell/organism. Next, the cell/organism suspension may be gelled by heating it up to a temperature higher than the sol-gel transition temperature of the hydrogel, to thereby produce a hydrogel with the cell/organism for fractionation substantially uniformly dispersed inside it. Next, the resulting hydrogel may be put in a field in which the physical property thereof selected from the electric field intensity, the magnetic field intensity, the light intensity, the temperature and the viscosity thereof continuously varies to thereby produce a gradation of the property in the inside of the hydrogel. With that, the cell/organism which have been substantially uniformly distributed inside the hydrogel according to the gradation may be then re-arranged in different sites in the hydrogel according to the difference in the taxis to the respective property. Next, the respective sites of the hydrogel may be cut out, and the cell/organism may be separated (fractionated, differentially separated, fractionally collected) in accordance with the difference in the taxis in the same manner as above.

According to the present inventors' findings, it is presumed that the hydrogel of the invention for separation (fractionated, differentially separated, fractionally collected) of cell/organism may utilize hydrophobic bonds in at least a part of the crosslinks therein. Of various physical bonds, hydrophobic bonds are only one type that may be strengthened with the increase in the ambient temperature. Using such hydrophobic bonds as crosslinking bonds makes it possible to produce the hydrogel favorably used in the invention, which is in a sol state at a low temperature and which gels at a high temperature. Changing the hydrophobic bonding force in the crosslinking point in the hydrogel makes it possible to change the sol-gel transition temperature of the hydrogel. Preferably, the sol-gel transition temperature of the hydrogel of the invention is higher 0°C but not higher than 45°C. For example, the physical property of the hydrogel make it possible to carry out the step of embedding cell, organisms, microorganism, tissues or organs into the hydrogel and collecting them from the hydrogel, not substantially causing any thermal damage or enzyme-based damage to them.

As opposed to this, since the crosslinking in an agar gel heretofore used for culture of cell, organisms or tissues (this shows a positive temperature dependency of solubility) is formed mainly by a crystalline structure, the bonding force therein is strong and the temperature at which the gel changes into a sol state is about 95°C and is extremely higher than a physiological temperature range (generally, from 0°C to 40°C). Therefore, it is impossible to embed cells, organisms, microorganism, tissues or organs into such an agar gel and to collect them from it. Regarding a conventional alginic acid gel (this shows a positive temperature dependency of solubility), the crosslinking is formed in a mode of ionic bonding therein, and therefore its bonding force is strong. Accordingly, it is difficult to change the gel into a sol state under a physiological condition, and it is therefore impossible to embed cell, organisms or the like into the gel and to collect them from it. Further, regarding conventional collagen and gelatin gels (these exhibit a positive temperature dependency of solubility), the crosslinking therein is formed as a crystalline structure or in a mode of ionic bonding. Therefore, the gels require enzyme such as collagenase or gelatinase for changing them into a sol state. Accordingly, it is difficult to collect cells, organisms, tissues or organs from such gels under a physiological condition (since using collagenase or gelatinase causes damage to biological tissues due to its enzymatic reaction).

On the other hand, another important property of the hydrogel of the invention is that cells, organisms, microorganisms, tissues or organs can move (in some degree) in the hydrogel. In order to get the above-mentioned physical property, it is indispensable that the crosslinking point bond of the three-dimensional network structure of the hydrogel is not too strong. In general, when the bonding energy of the crosslinking point of the three-dimensional network of a hydrogel is represented by $\Delta F$, then crosslinking point life ($\tau$) may be represented by the following formula:

$$\tau = \tau_0 \exp (\Delta F/KT)$$

Regarding the hydrogel having a three-dimensional network structure of which the crosslinking point life is $\tau$, the crosslinking point of the hydrogel is in a bonded condition to the action of the mode having a frequency higher than $1/\tau$ (sec$^{-1}$), or that is, the hydrogel is in the form of a crosslinked structure in that condition; but the crosslinking point of the hydrogel is in a non-bonded condition to the action of the mode having a frequency lower than $1/\tau$ (sec$^{-1}$), or that is, the hydrogel is liquid not having a crosslinked structure in that condition. This means that the hydrogel behaves as a solid to an extremely high frequency mode but behaves as a liquid to an extremely low frequency mode. Accordingly, the hydrogel behaves as a solid to the action occurring in transporting the hydrogel with cell, organisms, microorganisms, tissues or the like embedded therein, or in cutting it (in general, the frequency of the action is high, exceeding about $10^{-2}$ sec$^{-1}$ order), while it behaves as a liquid to the slow action of migration or proliferation that has a low frequency of less than about $10^{-4}$ sec$^{-1}$ order. Therefore, cells, organisms, tissues or the like may move in the hydrogel in accordance with the taxis.

Preferably, the bonding energy of the crosslinking points that form the three-dimensional network structure having the above-mentioned property is on the same level as that of the thermal energy (RT) in a physiological temperature range (0°C to 40°C). A three-dimensional network structure formed by bonding due to a dispersive force having a bonding energy of a few kcal/mol, hydrogen bonding or hydrophobic bonding is favorably usable as the hydrogel of the invention. A three-dimensional network structure to be formed by crosslinking in a mode of covalent bonding, crystalline structure or ionic bonding that has a much higher bonding energy level of from tens to hundreds kcal/mol than that of the former network structure is unsuitable to the hydrogel of the invention.

As so mentioned hereinabove, since the three-dimensional network structure, or that is, the hydrogel formed by hydrophobic bonding has the property that its hydrophobic bonding is strengthened with the increase in the ambient temperature, it is in a sol state at a low temperature and gels at a high temperature. Accordingly, the sol-gel transition temperature dependence of the hydrogel of the type is opposite to that of any other hydrogel that utilizes other bonding such as hydrogen bonding or bonding by dispersive force. The physical property of the hydrogel that utilizes hydrophobic bonding enable to embed cell/organism in a low-temperature sol state of the hydrogel, and therefore the hydrogel of the type is more favorably usable as the hydrogel for fractionation of cell/organism of the invention than conventional hydrogels, because the hydrogel of the former type can evade thermal damage in embedding cell/organism therein. Further, since the phase transition of the hydrogel that utilizes hydrophobic bonding is thermally reversible, the gel can

EP 1 595 944 A1

be dissolved at a low temperature when the gel is removed from the cell/organism embedded therein, and the cell/organism can be readily isolated from the gel not causing thermal damage to them.

BRIEF DESCRIPTION OF THE DRAWINGS

Fig. 1 is a schematic perspective view showing a preferred embodiment of the invention.
Fig. 2 is a schematic perspective view showing another preferred embodiment of the invention.

BEST MODE FOR CARRYING OUT THE INVENTION

The invention is described more concretely with reference to the drawings if desired. In the following description, "part" and "%" indicating an amount ratio is by mass unless otherwise specifically indicated.

(Hydrogel for separating cell/organism)

The hydrogel of the invention for separation (fractionation, differential separation, fractional collection) of cell/organism contains, as its aqueous solution, a hydrogel-forming polymer having a sol-gel transition temperature, and it shows thermo-reversible sol-gel transition of such that it is in a sol state at lower temperatures and gels at higher temperatures. In the invention, the term "cell/organism" means "cell and/or organisms", including any cell and cell aggregates associated with or derived from organisms (plants and animals) that contain one or more cell, so far as they exhibit some taxis to a physiological substance. In the invention, the morphology of the cell and cell aggregates is not specifically defined, including, for example, unicellular and multicellular organisms or organs, microorganisms, sperms, eggs.

(Separation)

In the invention, the term "separation" means that the above-mentioned cell/organism is differentiated in point of the spatial position based on the difference in any taxis they have. In the invention, the morphology for "differentiation in point of the spatial position" is not specifically defined.
In a different viewpoint, the term "separation" as referred to herein is meant to indicate any separation operation capable of realizing some selectivity in the position of cell/organism by utilizing the selective movement of cell/organism inside a gel and/or between the inside a gel and a gel-external environment based on the chemotaxis of the cell/organism or based on the taxis thereof to varying physical property around them. The "separation" includes, for example, fractionation, differential separation, fractional collection.

(Sol-gel transition temperature)

In the present invention, the definition and measurement of the "sol state," "gel state," and "sol-gel transition temperature" are based on the definition and method described in a publication (H. Yoshioka et al., Journal of Macromolecular Science, A31(1), 113 (1994)).
That is, the dynamic elastic modulus of a sample at an observed frequency of 1 Hz is determined by gradually shifting the temperature from a low temperature side to a high temperature side (1°C/1 min). In this measurement, the sol-gel transition temperature is defined as a temperature at which the storage elastic modulus (G', elastic term) of the sample exceeds the loss elastic modulus (G", viscous term). In general, the sol state is defined as a state in which G" > G' is satisfied, and the gel state is defined as a state in which G" < G' is satisfied. For the measurement of such a sol-gel transition temperature, the following measuring conditions can preferably be used.

<Measuring conditions for dynamic and loss elastic moduli>

Measuring apparatus (trade name): Stress controlled-type rheometer (model: CSL-500, mfd. by Carri-Med Co.)
Concentration of sample solution (or dispersed liquid) (as a concentration of a "polymer compound having a sol-gel transition temperature"): 10% (by weight)
Amount of sample solution: about 0.8 g
Shape and size of cell for measurement: acrylic parallel disk (diameter: 4.0 cm), gap: 600 μm

Measurement frequency: 1 Hz
Stress to be applied: within linear region

In the present invention, the above sol-gel transition temperature may preferably be higher than 0 °C and not higher than 45 °C, more preferably, higher than 0 °C and not higher than 42 °C (particularly not lower than 4 °C and not higher than 40 °C) in view of the prevention of a thermal damage to cell or a tissue of a living organism.

The hydrogel material having such a preferred sol-gel transition temperature may easily be selected from specific compounds as described below, according to the above-mentioned screening method (method of measuring the sol-gel transition temperature).

In a sequence of operations wherein a cell/organism is subjected to separation (fractionation, differential separation, fractional collection) by using the hydrogel according to the present invention, it is preferred to set the above-mentioned sol-gel transition temperature ($\underline{a}$ °C) between the temperature at the time of the conducting separation (fractionation, differential separation, fractional collection) of cell/organism (b °C), and the temperature at the time of the cooling for the inoculation, mixing or recovery of the cell or tissue (c °C). In other words, the above-mentioned three kinds of temperatures of $\underline{a}$ °C, b °C and c °C may preferably have a relationship of b > $\underline{a}$ > c. More specifically, the value of (b - a) may preferably be 1 - 40 °C, more preferably 2 - 30 °C. On the other hand, the value of (a - c) may preferably be 1 - 40 °C, more preferably 2 - 30 °C..

(Cell selection capability)

It is desirable that the hydrogel favorably used in the invention has a cell-selecting capability $R/R_0$, as determined according to the measuring method mentioned below, of at least 2, more preferably at least 5, even more preferably at least 10, from a view point that the hydrogel of the type shows good cell selectivity. The cell selection capability $R/R_0$ may be determined as follows:

One grown of a hydrogel containing $10^{-6}$ M of fMLP and having an S/V (surface area/volume ratio of from 10 to 15 is contacted with 10 ml of a citrated human whole blood, at 37°C for 4 hours in a 14-ml disposable centrifugal tube, and the ratio of the number of leucocytes (L) to the number of erythrocytes (E) which entered the hydrogel, R = L/E, is measured. On the other hand, the ratio of the number of leucocytes ($L_0$) to the number of erythrocytes ($E_0$) in the citrated human whole blood which is previously measured, $R_0 = L_0/E_0$, is calculated. The obtained ratio, $R/R_0$ is the cell selection capability.

(Mobility of cell/organism in hydrogel)

In a viewpoint such that a cell/organism is freely movable in a hydrogel, it is preferred that the hydrogel according to the present invention shows a behavior in a solid-like manner toward a higher frequency, and that the hydrogel shows a behavior in a liquid-like manner toward a lower frequency. More specifically, the property of the hydrogel for the mobility of cell/organism may preferably be measured according to the following method.

(Method of measuring mobility of cell/organism in hydrogel)

The hydrogel according to the present invention comprising a hydrogel-forming polymer in a sol state (i.e., at a temperature lower than the sol-gel transition temperature) is poured into a test tube having an inside diameter of 1 cm, in an amount of the hydrogel-forming polymer solution corresponding to a volume of 1 mL as the resultant hydrogel. Then, the above test tube is left standing for 12 hours in a water bath which is controlled at a temperature which is sufficiently higher than the sol-gel transition temperature of the hydrogel (e.g., a temperature which is 10 °C higher than the sol-gel transition temperature), whereby the hydrogel material is converted into a gel state.

Then, when the test tube is turned upside down, there is measured the time (T) until the interface (meniscus) between the solution and air is deformed due to the weight of the solution per se. Herein, the hydrogel will show a behavior in a liquid-like manner toward a movement having a frequency lower than 1/T ($sec^{-1}$), and the hydrogel will show a behavior in a solid-like manner toward a movement having a frequency higher than 1/T ($sec^{-1}$). In the case of the hydrogel according to the present invention, T may preferably be 1 minute to 24 hours, more preferably 5 minutes to 10 hours.

(Steady-state flow kinematic viscosity)

Alternatively, the gel property of the hydrogel according to the present invention may preferably be determined by measuring the steady-state flow kinematic viscosity thereof. For example, the steady-state flow kinematic viscosity η (eta) may be measured by using a creep experiment.

In the creep experiment, a predetermined shear stress is imparted to a sample, and a time-dependent change in the resultant shear strain is observed. In general, in the creep behavior of viscoelastic material, the shear rate is changed with the elapse of time in an initial stage, but thereafter shear rate becomes constant. The Steady-state flow kinematic viscosity η is defined as the ratio of the shear stress and the shear rate at this time. This Steady-state flow kinematic viscosity can also be called Newtonian viscosity. However, it is required that the Steady-state flow kinematic viscosity is determined in the linear region wherein the viscosity little depends on the shear stress.

In a specific embodiment of the measuring method, a stress-controlled type viscoelasticity-measuring apparatus (model: CSL-500, mfd. by Carri-Med Co., USA) is used as the measuring apparatus, and an acrylic disk (having a diameter of 4 cm) is used as the measuring device, and the resultant creep behavior (delay curve) is measured for at least five minutes with respect to a sample having a thickness of 600 μm. The sampling time is once per one second for the initial 100 seconds, and once per ten seconds for subsequent period.

When the shear stress (stress) to be applied to the sample is determined, the shear stress should be set to a minimum value such that a displacement angle of $2 \times 10^{-3}$ rad or more is detected, when such a shear stress is loaded for ten seconds counted from the initiation of the measurement. When the resultant data is analyzed, at least 20 or more measured values are adopted with respect to the measurement after five minutes. The hydrogel according to the present invention may preferably have an η of $5 \times 10^3$-$5 \times 10^6$ Pa·sec, more preferably $8 \times 10^3$-$2 \times 10^6$ Pa·sec, particularly, not less than $1 \times 10^4$ Pa·sec and not more than $1 \times 10^6$ Pa·sec, at a temperature which is about 10 °C higher than the sol-gel transition temperature.

When the above η is less than $5 \times 10^3$ Pa·sec, the fluidity becomes relatively high even in a short-time observation, and the suppression of free diffusion effect on cell/organism by the gel is liable to be insufficient, or the provision of a concentration gradient of a physiologically active substance by the gel is liable to be insufficient in some cases. On the other hand, when η exceeds $5 \times 10^6$ Pa·sec, the tendency that the gel shows little fluidity even in a long-time observation is strengthened, and difficulty in the movement of the cell/organism due to the taxis is increased. In addition, when η exceeds $5 \times 10^6$ Pa·sec, the possibility that the gel shows a fragility is strengthened, and the tendency of brittle fracture that, after a slight pure elastic deformation, the gel is easily destroyed at a stroke is strengthened.

(Dynamic elastic modulus)

Alternatively, the gel property of the hydrogel according to the present invention may preferably be determined by measuring the dynamic elastic modulus thereof. Provided that when a strain $\gamma$ (t) = $\gamma_0$ cos ωt (t is time) having an amplitude $\gamma_0$, number of vibrations of ω/2π to the gel, a stressσ (t) = $\sigma_0$cos (ωt+ δ) having a constant stress of $\sigma_0$ and a phase difference of δ is obtained. When | G | = $\sigma_0$/ $\gamma_0$, the ratio (G"/G') between the dynamic elastic modulus G '(ω) = |G| cos δ and the loss elastic modulus G "(ω) = |G| sin δ is an indicator showing the degree of gel property.

The hydrogel according to the present invention behaves as a solid toward a stress of ω/2π = 1 Hz (corresponding to a fast movement), and behaves as a liquid toward a stress of ω/2π =$10^{-4}$ Hz (corresponding to a slow movement). More specifically, the hydrogel according to the present invention may preferably show the following property (with respect to the details of the method of measuring elastic modulus, e.g., literature: "Modern Industrial Chemistry" (Kindai Kyogyo Kagaku) No. 19, edited by Ryohei Oda, et al., Page 359, published by Asakura Shoten, 1985 may be referred to).

In the case of ω/2π = 1 Hz (number of vibrations at which the gel behaves as a solid), the ratio (G "/G ')$_s$ = (tan δ)$_s$ may preferably be less than 1 (preferably 0.8 or less, particularly, 0.5 or less).

In the case of ω/2π =$10^{-4}$ Hz (number of vibrations at which the gel behaves as a liquid), the ratio (G "/G ')$_L$ = (tan δ)$_L$ may preferably be 1 or more (preferably 1.5 or more, particularly, 2 or more).

The ratio {(tan δ)$_s$/ (tan δ)$_L$} between the above (tan δ)$_s$ and (tan δ)$_L$ may preferably be less than 1 (mire preferably 0.8 or less, particularly, 0.5 or less).

<Measurement conditions>

    Concentration of hydrogel-forming polymer: about 8 mass %
    Temperature: a temperature which is about 10 °C higher than the sol-gel transition temperature of the carrier
    Measuring apparatus: Stress controlled-type rheometer (model: CSL-500, mfd. by Carri-Med Co., USA)

(Hydrogel-forming polymer)

The hydrogel-forming polymer usable for the hydrogel according to the present invention is not particularly limited, as long as the polymer shows the above-mentioned thermo-reversible sol-gel transition (that is, as long as it has a sol-gel transition temperature). It is preferable to achieve a preferred sol-gel transition temperature by adjusting the cloud point of a plurality of blocks having a cloud point and the cloud point of a hydrophilic block in the hydrogel-forming polymer, the compositions, hydrophobicity or hydrophilicity of both types of blocks, and/or the molecular weights, in view of easy exhibition of a preferred sol-gel transition within the physiological temperature (about 0°C to 42°C).

As specific examples of the polymer such that an aqueous solution thereof has a sol-gel transition temperature, and it reversibly assumes a sol state at a temperature lower than the sol-gel transition temperature., there have been known, e.g., polyalkylene-oxide block copolymer represented by block copolymers comprising polypropylene oxide portions and polyethylene oxide portions; etherified (or ether group-containing) celluloses such as methyl cellulose and hydroxypropyl cellulose; chitosan derivatives (K.R. Holme. et al. Macromolecules, 24, 3828 (1991)), etc.

In addition, there has been developed a gel utilizing Pluronic F-127 (trade name, mfd. by BASF Wyandotte Chemical Co.) comprising a polypropylene oxide portion and polyethylene oxide portions bonded to the both terminals thereof.

It is known that a high-concentration aqueous solution of the above Pluronic F-127 is converted into a hydrogel at a temperature of not lower than about 20 °C, is converted into an aqueous solution at a temperature lower than this temperature. However, this material can assume a gel state only at a high concentration of not lower than about 20 mass %. In addition, even when such a gel having a high concentration of not lower than about 20 mass % is maintained at a temperature higher than the gel-forming temperature, the gel is dissolved when water is further added thereto. In addition, since the molecular weight of the Pluronic F-127 is relatively low, and it shows an extremely high osmotic pressure at a high concentration of not less than about 20 mass. %, and simultaneously the Pluronic F-127 may easily permeate the cell membranes, whereby the Pluronic F-127 can adversely affect cell or tissue of an organism.

On the other hand, in the case of an etherified cellulose represented by methyl cellulose, hydroxypropyl cellulose, etc., the sol-gel transition temperature thereof is as high as about 45 °C or higher (N. Sarkar, J. Appl. Polym. Science, 24, 1073, (1979)). On the other hand, the fractionation and differential separation of cell/organism is usually conducted in the neighborhood of 37 °C or a lower temperature, and therefore, it is actually difficult to use such an etherified cellulose for the purpose of the fractionation and differential separation of cell/organism.

As described above, when a conventional polymer having a sol-gel transition temperature in an aqueous solution thereof, and reversibly assuming a sol state at a temperature lower than the above transition temperature is simply used, the following problems are posed:

(1) If the polymer is once converted into a gel state at a temperature higher than the sol-gel transition temperature, the resultant gel is dissolved when water is further added thereto;
(2) The polymer has a sol-gel transition temperature higher than the temperature for the fractionation or differential separation of cell/organism (in the neighborhood of 37 °C or lower), and therefore the polymer assumes a sol state at the temperature for the fractionation or differential separation;
(3) It is necessary to increase the concentration of the polymer in an aqueous solution thereof to an extremely high value, in order to convert the polymer into a gel state; etc.

On the other hand, according to the present inventor's investigation, it has been found that the above problem can be solved by constituting the base material for the separation (such as fractionation, differential separation, and fractional collection) of cell/organism according to the present invention by use of a polymer having a sol-gel transition temperature of higher than 0 °C and not higher than 42 °C (e.g., a polymer which comprises a plurality of polymer chains having a cloud point, and a hydrophilic polymer chain block which has been bonded thereto; and an aqueous solution of which has a sol-gel transition temperature, and reversibly assumes a sol state at a temperature lower than the sol-gel transition temperature).

(Preferred hydrogel-forming polymers)

The hydrogel-forming polymer preferably usable as the base material for the separation (such as fractionation, differential separation, and fractional collection) of cell/organism according to the present invention may preferably comprise a combination of plural hydrophobic blocks having a cloud point, and a hydrophilic block bonded thereto. The presence of the hydrophilic block is preferred in view of the provision of the water-solubility of the hydrogel material at a temperature lower than the sol-gel transition temperature. The presence of the plural hydrophobic block having a cloud point is preferred in view of the conversion of the hydrogel material into a gel state at a temperature higher than the sol-gel transition temperature. In other words, the blocks having a cloud point become water-soluble at a temperature lower than the cloud point, and are converted into a water-insoluble state at a temperature higher than the cloud point, and therefore these blocks function as crosslinking points constituted by hydrophobic bonds for forming a gel at a temperature higher than the cloud point. That is, the cloud point based on the hydrophobic bonds corresponds to the above-mentioned sol-gel transition temperature of the hydrogel.

However, it is not always necessary that the cloud point corresponds to the sol-gel transition temperature. This is because the cloud point of the above-mentioned "blocks having a cloud point" is generally influenced by the bonding between the hydrophilic block and the blocks having a cloud point.

The hydrogel to be use in the present invention utilizes a property of hydrophobic bonds such that they are not only strengthened along with an increase in temperature, but also the change in the hydrophobic bond strength is reversible with respect to the temperature. In view of the formation of plural crosslinking points in one molecule, and the formation of a gel having a good stability, the hydrogel-forming polymer may preferably have a plurality of "blocks having cloud point".

On the other hand, as described above, the hydrophilic block in the hydrogel-forming polymer has a function of causing the hydrogel-forming polymer to be changed into a water-soluble state at a temperature lower than sol-gel transition temperature. The hydrophilic block also has a function of providing the state of an aqueous (or water-containing) gel, while preventing the aggregation and precipitation of the hydrogel material due to an excess increase in the hydrophobic binding force at a temperature higher than the transition temperature.

(Plural blocks having cloud point)

The plural block having a cloud point may preferably comprise a polymer block which shows a negative solubility-temperature coefficient with respect to water. More specifically, such a polymer may preferably be one selected from the group consisting of: polypropylene oxide, copolymers comprising propylene oxide and another alkylene oxide, poly N-substituted acrylamide derivatives, poly N-substituted methacrylamide derivatives, copolymers comprising an N-substituted acrylamide derivative and an N-substituted methacrylamide derivative, polyvinyl methyl ether, and partially-acetylated product of polyvinyl alcohol.

It is preferred that the above polymer (block having a cloud point) has a cloud point of higher than 4 °C and not higher than 45 °C, in view of the provision of a polymer (compound comprising a plurality of blocks having a cloud point, and a hydrophilic block bonded thereto) to be used in the present invention having a sol-gel transition temperature of higher than 4 °C and not higher than 40°C.

It is possible to measure the cloud point, e.g., by the following method. That is, an about 1 wt.%-aqueous solution of the above polymer (block having a cloud point) is cooled to be converted into a transparent homogeneous solution, and thereafter the temperature of the solution is gradually increased (temperature increasing rate: about 1 °C/min.), and the point at which the solution first shows a cloudy appearance is defined as the cloud point.

Specific examples of the poly N-substituted acrylamide derivatives and poly N-substituted methacrylamide derivatives are described below.

Poly-N-acryloyl piperidine
Poly-N-n-propyl methacrylamide
Poly-N-isopropyl acrylamide
Poly-N,N-diethyl acrylamide
Poly-N-isopropyl methacrylamide
Poly-N-cyclopropyl acrylamide
Poly-N-acryloyl pyrrolidine
Poly-N,N-ethyl methyl acrylamide
Poly-N-cyclopropyl methacrylamide
Poly-N-ethyl acrylamide

The above polymer may be either a homopolymer or a copolymer comprising a monomer constituting the above polymer and "another monomer". The "another monomer" to be used for such a purpose may be either a hydrophilic monomer, or a hydrophobic monomer. In general, when copolymerization with a hydrophilic monomer is conducted, the resultant cloud point may be increased. On the other hand, when copolymerization with a hydrophobic monomer is conducted, the resultant cloud point may be decreased. Accordingly, a polymer having a desired cloud point (e.g., a cloud point of higher than 4 °C and not higher than 45°C) may also be obtained by selecting such a monomer to be used for the copolymerization.

(Hydrophilic monomer)

Specific examples of the above hydrophilic monomer may include: N-vinyl pyrrolidone, vinyl pyridine, acrylamide, methacrylamide, N-methyl acrylamide, hydroxyethyl methacrylate, hydroxyethyl acrylate, hydroxymethyl methacrylate, hydroxymethyl acrylate, methacrylic acid and acrylic acid having an acidic group, and salts of these acids, vinyl sulfonic acid, styrenesulfonic acid, etc., and derivatives having a basic group such as N,N-dimethylaminoethyl methacrylate, N,N-diethylaminoethyl methacrylate, N,N-dimethylaminopropyl acrylamide, salts of these derivatives, etc. However, the hydrophilic monomer to be usable in the present invention is not restricted to these specific examples.

(Hydrophobic monomer)

On the other hand, specific examples of the above hydrophobic monomer may include: acrylate derivatives and methacrylate derivatives such as ethyl acrylate, methyl methacrylate, and glycidyl methacrylate; N-substituted alkyl methacrylamide derivatives such as N-n-butyl methacrylamide; vinyl chloride, acrylonitrile, styrene, vinyl acetate, etc. However, the hydrophobic monomer to be usable in the present invention is not restricted to these specific examples.

(Hydrophilic block)

On the other hand, specific examples of the hydrophilic block to be combined with (or bonded to) the above-mentioned block having a cloud point may include: methyl cellulose, dextran, polyethylene oxide, polyvinyl alcohol, poly N-vinyl pyrrolidone, polyvinyl pyridine, polyacrylamide, polymethacrylamide, poly N-methyl acrylamide, polyhydroxymethyl acrylate, polyacrylic acid, polymethacrylic acid, polyvinyl sulfonic acid, polystyrene sulfonic acid, and salts of these acids; poly N,N-dimethylaminoethyl methacrylate, poly N,N-diethylaminoethyl methacrylate, poly N,N-dimethylaminopropyl acrylamide, and salts of these, etc.

The process for combining the above block having a cloud point with the hydrophilic block is not particularly limited. For example, it is possible to conduct such a combination by introducing a polymerizable functional group (such as acryloyl group) into either one of the above blocks, and copolymerizing with the resultant product a monomer capable of providing the other block. Alternatively, it is also possible to obtain a combination product of the above block having a cloud point with the hydrophilic block by copolymerizing a monomer capable of providing the block having a cloud point with a monomer capable of providing the hydrophilic block.

In addition, the block having a cloud point and the hydrophilic block may also be combined or bonded with each other by preliminarily introducing reactive functional groups (such as hydroxyl group, amino group, carboxyl group, and isocyanate group) into both kinds of the blocks, and combining these blocks by using a chemical reaction. At this time, it is usual to introduce a plurality of reactive functional groups into the hydrophilic block.

Further, the polypropylene oxide having a cloud point and the hydrophilic block may be combined or bonded with each other by repetitively subjecting polypropylene oxide and a monomer constituting the above "other water-soluble block" (such as ethylene oxide) to a stepwise or consecutive polymerization, to thereby obtain a block copolymer comprising polypropylene oxide and a water-soluble block (such as polyethylene oxide) combined therewith.

Such a block copolymer may also be obtained by introducing a polymerizable group (such as acryloyl group) into the terminal of polypropylene oxide, and then copolymerizing therewith a monomer constituting the hydrophilic block.

Further, a polymer usable in the present invention may be obtained by introducing a functional group which is reactive in a bond-forming reaction with the terminal functional group of polypropylene oxide (such as hydroxyl group) into a hydrophilic block, and reacting the resultant hydrophilic block and the polypropylene oxide. In addition, a hydrogel-forming polymer usable in the present invention may be obtained by connecting materials such as one comprising polypropylene glycol and polyethylene glycol bonded to both terminals thereof (such as Pluronic F-127; trade name, mfd. by Asahi Denka Kogyo K.K.).

In an embodiment of the present invention wherein the hydrogel-forming polymer comprises a block having a cloud point, at a temperature lower than the cloud point, the polymer may completely be dissolved in water so as to assume a sol state, since the above-mentioned "block having a cloud point" present in the polymer molecule is water-soluble together with the hydrophilic block. However, when a solution of the above polymer is heated up to a temperature higher than the cloud point, the "block having a cloud point" present in the polymer molecule becomes hydrophobic so that separate molecules of the polymer are associated or aggregated with each other due to a hydrophobic interaction.

On the other hand, the hydrophilic block is water-soluble even at this time (i.e., even when heated up to a temperature higher than the cloud point), and therefore, the polymer according to the present invention in water is formed into a hydrogel having a three-dimensional network structure wherein hydrophobic association portions between the blocks having a cloud point constitute the crosslinking points. The resultant hydrogel is again cooled to a temperature lower than the cloud point of the "block having a cloud point" present in the polymer molecule, the block having a cloud point becomes water-soluble and the above crosslinking points due to the hydrophobic association are released or liberated so that the hydrogel structure disappears, whereby the polymer according to the present invention is again formed into a complete aqueous solution. In the above-described manner, the sol-gel transition in the polymer according to the present invention is based on the reversible hydrophilic-hydrophobic conversion in the block having a cloud point present in the polymer molecule at the cloud point, and therefore the transition has a complete reversibility in accordance with a temperature change.

(Solubility of gel)

As described above, the hydrogel-forming polymer according to the present invention comprising at least a polymer having a sol-gel transition temperature in an aqueous solution thereof, substantially shows a water insolubility at a temperature (d °C) higher than the sol-gel transition temperature, and reversibly shows water solubility at a temperature (e °C) lower than the sol-gel transition temperature.

The above-mentioned temperature (d °C) may preferably be a temperature which is at least 1 °C, more preferably at least 2 °C (particularly preferably, at least 5 °C) higher than the sol-gel transition temperature. Further, the above-mentioned "substantial water insolubility" may preferably be a state wherein the amount of the above polymer to be dissolved in 100 ml of water at the above temperature (d °C) is 5.0 g or less (more preferably 0.5 g or less, particularly preferably 0.1 g or less).

On the other hand, the above-mentioned temperature (e °C) may preferably be a temperature which is at least 1 °C, more preferably at least 2 °C (particularly preferably, at least 5 °C) lower than the sol-gel transition temperature, in terms of the absolute values of these temperatures. Further, the above-mentioned "water solubility" may preferably be a state wherein the amount of the above polymer to be dissolved in 100 ml of water at the above temperature (e °C) is 0.5 g or more (more preferably 1.0 g or more). The above "to show a reversible water solubility" refers to a state wherein an aqueous solution of the above hydrogel-forming polymer shows the above-mentioned water solubility at a temperature lower than the sol-gel transition temperature, even after the polymer is once formed into a gel state (at a temperature higher than the sol-gel transition temperature).

A 10%-aqueous solution of the above polymer may preferably show a viscosity of 10 - 3,000 centipoises, more preferably 50 - 1,000 centipoises at 5 °C. Such a viscosity may preferably be measured, e.g., under the following measurement conditions:

Viscometer: Stress-controlled type rheometer (model: CSL-500, mfd. by Carri-Med Co., USA)
Rotor diameter: 60 mm
Rotor configuration: Parallel-plate type
Measurement frequency: 1 Hz (hertz)

Even when the an aqueous solution of the hydrogel-forming polymer according to the present invention is formed into a gel state at a temperature higher than the sol-gel transition temperature, and thereafter the resultant gel is immersed in a large amount of water, the gel is not substantially dissolved in water. For example, such a characteristic of the above the above base material for the separation (such as fractionation, differential separation, and fractional collection) of cell/organism may be confirmed in the following manner.

More specifically, 0.15 g of the hydrogel-forming polymer according to the present invention is dissolved in 1.35 g of distilled water at a temperature lower than the above sol-gel transition temperature (e.g., under cooling with ice) to thereby prepare a 10 mass %-aqueous solution. Then, the resultant solution is poured into a plastic Petri dish having a diameter of 35 mm, then the dish is warmed up to a temperature of 37 °C to form a gel having a thickness of about 1.5 mm in the dish, and the total weight of the Petri dish (f gram) containing the gel is measured. Then, the entirety of the Petri dish containing the gel is left standing in 250 ml of water at 37 °C for 10 hours, and thereafter the total weight of the Petri dish (g gram) containing the gel is measured, to thereby determine whether the gel has been dissolved from the gel surface or not. At this time, in the hydrogel-forming polymer according to the present invention, the ratio of weight decrease in the gel, i.e., the value of {(f-g)/f} may preferably be 5.0 % or less, more preferably 1.0 % or less (particularly preferably 0.1 % or less).

Even when an aqueous solution of the hydrogel-forming polymer according to the present invention was converted into a gel state at a temperature higher than the sol-gel transition temperature, and then the resultant gel was immersed in a large amount (about 0.1 - 100 times larger than the gel, by volume ratio), the gel was not dissolved for a long period of time. Such a property of the polymer to be used in the present invention may be achieved, e.g., by the presence of at least two (a plurality of) blocks having a cloud point in the polymer molecule.

On the contrary, according to the present inventors' experiments, in a case where a similar gel was formed by using the above-mentioned Pluronic F-127 comprising polypropylene oxide and polyethylene oxide bonded to both terminals thereof, the resultant gel was completely dissolved when the gel is left standing in water for several hours.

In order to suppress the cytotoxicity of a non-gel state to a low level as completely as possible, it is preferred to use a hydrogel-forming polymer which can be converted into a gel state at a concentration of 20% or less (more preferably 15% or less, particularly 10% or less) in terms of the concentration of the polymer based on water, i.e., {(polymer)/(polymer + water)}×100 (%).

(Physiologically active substance)

The physiologically active substance as referred to in the invention is meant to indicate a substance having affinity, reactivity or inductivity to cell and organisms. For example, it includes tactic factor (chemotactic factor), antibody, cytokine and its receptor, cell-adhesion factor.

Chemotaxis is a property of cell or microorganism, meaning that, after stimulated by a difference in the concentration of a chemical substance (chemotactic factor), cell or microorganism gather or escape in accordance with the concentration gradient. Many microorganism and cell such as leucocytes, cancer cell, sperms have the property of chemotaxis, and microorganism, cell and organisms have the ability to recognize a chemotactic factor specific to them. For example, typical chemotactic factors are an immunoglobulin-derived factor acting on neutrophils and macrophages; complement-derived factors C3a, C5a, N-formyl-Met-Lew-Phe acting on neutrophils; a lymphocyte-derived factor, lymphokine acting on macrophages; a peptide-like factor, ecalectin acting on eosinophilic leucocytes. These are chemotactic factors to immunity-associated cell relating to allergic reaction.

As so mentioned hereinabove, recently it has become considered that various cell growth factors such as a blood vessel endothelial cell growth factor associated with induction and regeneration of blood vessels, and a neurocyte growth factor associated with induction and regeneration of neuropil may be chemotactic factors. It is known that blood vessel systems may be induced and regenerated through oxygen concentration gradient, and it may be said that oxygen is a negative tactic factor. In addition, it has become known that a chemotactic factor participates in the metastasis of cancer cell. As in the above, it may be considered that many immunity-related or cancer-related drugs that directly act on cell will have chemotactic activity.

(Physicotactic factor)

Physicotaxis is a property of cell or microorganism, meaning that, after stimulated by the difference in the intensity of a physical factor, cell or microorganism gather or escape in accordance with the intensity difference. Many microorganism and cell have the property of physicotaxis, and they have the ability to recognize a physicotactic factor specific to them. For example, typical physicotactic factors are electric field, magnetic field, gravity field, light intensity, temperature, viscosity.

(Tactic factor)

In the invention, the tactic factor preferably has a cell-attracting capability $N/N_0$, as determined according to the measuring method mentioned below for the targeted cell, of at least 1.2, more preferably at least 2, even more preferably at least 10. The cell-attracting capability may be measured, for example, as follows:

One gram of the hydrogel-forming polymer (TGP-5) of the invention obtained in Production Example 7 mentioned below is dissolved in 9 g of physiological saline containing a tactic factor, at 4°C to prepare an aqueous 10 wt. % solution of the hydrogel-forming polymer of the invention. In this, the concentration of the tactic factor in the aqueous hydrogel-forming polymer solution must fall within a range within which the solution can attract the targeted cell. In general, it is from $10^{-6}$ M to $10^{-5}$ M. One gram of the aqueous solution is heated up to 37°C to form a hydrogel having a surface area of from 10 to 15 $cm^2$ (S/V ratio, from 10 to 15). Concretely, for example, the aqueous solution is filled into a one-ml syringe equipped with a 23G needle, cooled to 4°C, and then extruded out into 100 ml of physiological saline at 37°C, taking from 5 to 10 seconds. The string-like hydrogel obtained in this stage has a diameter of about 3 mm and a length of about 14 cm, therefore having a surface area of about 13 $cm^2$. One g of the hydrogel containing from $10^{-6}$ M to $10^{-5}$ M of the above tactic factor and having an S/V ratio of from 10 to 15 is contacted with a suspension of the targeted cell (number of cell: $10^6$ cell/ml) in a 14-ml disposable centrifugal tube, and gently rotated and stirred at 37°C for 4 hours. With the hydrogel left as such, the cell suspension is removed through decantation, and 10 ml of physiological saline is newly added to it at 37°C to thereby wash and remove the cell having adhered to the surface of the hydrogel. This washing operation is repeated three times, and then the hydrogel is cooled to 4°C and dissolved, and the cell number, N, of the cell suspending in the hydrogel is counted. On the other hand, the hydrogel of the invention not containing the tactic factor is tested in the same manner as above, and the cell number, $N_0$, of the cell suspending in the hydrogel is counted. The cell-attracting capability of the tactic factor, $N/N_0$ is thus obtained.

(Apparatus and method for separating cell/organism on the basis of the chemotaxis)

The apparatus and the method for separating (fractionating, differentially separating or fractionally collecting) cell on the basis of the chemotaxis by the use of the hydrogel of the invention may be grouped, for example, into the following three types, based on the process for making cell/organism suspend in the hydrogel. 1) A method comprising keeping an aqueous solution that contains a physiologically active substance separated from a suspension of cell/organism for fractionation via a hydrogel put therebetween, to thereby produce a concentration gradient of the physiologically active substance in the hydrogel, and transferring the cell/organism from the cell/organism suspension into the hydrogel based on the chemotaxis induced by the concentration gradient. 2) A method comprising producing a hydrogel with cell/organism substantially uniformly dispersed inside it, then contacting the hydrogel with an aqueous solution containing a physiologically active substance at a temperature higher than the sol-gel transition temperature of the hydrogel to thereby transfer the physiologically active substance into the hydrogel so as to produce a concentration gradient of the physiologically active substance in the hydrogel, and re-arranging the cell/organism which have been substantially uniformly distributed inside the hydrogel according to the concentration gradient in different sites in the hydrogel according to the difference in the chemotaxis. 3) A method comprising uniformly mixing a physiologically active substance in an aqueous solution of a hydrogel-forming polymer in a sol state, heating the sol-phase mixture up to a temperature higher than the sol-gel transition temperature of the mixture to thereby produce a hydrogel having a predetermined shape, and then contacting it with a suspension of cell/organism at a temperature higher than the sol-gel transition temperature so as to transfer the cell/organism into the predetermined shape-having hydrogel.

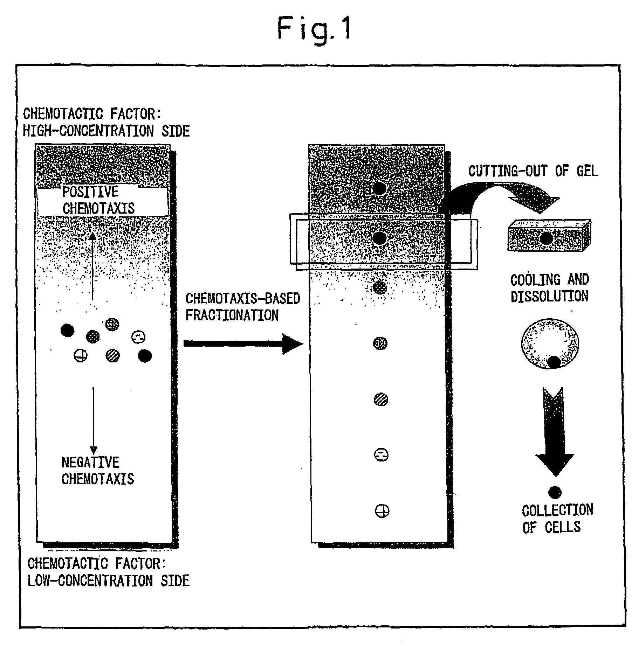

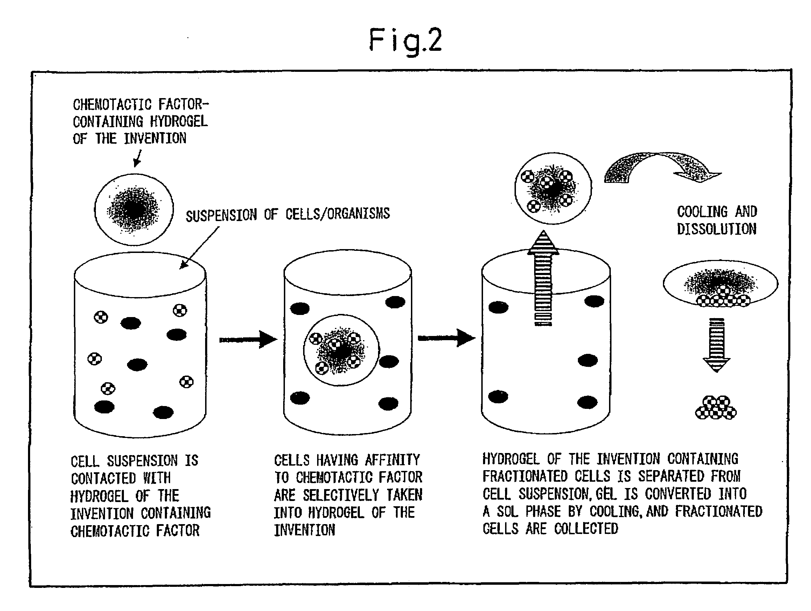

Fig. 1 schematicly shows the embodiment of the invention according to the method 1) and the method 2); and Fig. 2 schematicly shows the embodiment according to the method 3). Any of the above-mentioned methods may be employed, as suitably selected depending on the type of the targeted cell/organism. The shape of the hydrogel may also be suitably selected in accordance with the object thereof, and the hydrogel may have any shape of columns, discs, rectangular parallelepipeds, sphere, string, fiber, flake, plates, film or indeterminate shape.

In particular, when the method 3) is employed, it is desirable that the predetermined shape to be imparted to the hydrogel has a large surface area per the unit volume in order to increase the contact frequency between the physiologically active substance (tactic factor)-containing hydrogel and the cell in the cell suspension disposed around it. Preferably, the hydrogel has any shape of sphere, string, fiber, flake, plates, film or indeterminate shape. Also preferably, the predetermined shape to be imparted to the hydrogel has a ratio of surface area (S)/volume (V) of at least 10 (cm$^{-1}$), more preferably at least 30 (cm$^{-1}$), even more preferably at least 60 (cm$^{-1}$).

In addition, when the method 3) is employed, it is desirable to stir or circulate the cell suspension in order to increase the contact frequency between the hydrogel and the cell in the cell suspension disposed around it. A large number of the pairs of the hydrogel and the cell suspension disposed around it may be processed, as arranged in parallel. In this case, the tactic factor concentration in the hydrogel may be varied in each pair, or the contact time between the hydrogel and the cell suspension may also be varied in each pair, and the targeted cell/organism may be thereby fractionated and collected.

Concrete embodiments of the invention are described below.

(Fractionation and collection of leucocytes)

fMLP (N-formyl-methionyl-leucyl-phenylalanine: molecular weight 437.6, chemotactic peptide) and LPS (lipopolysaccharide) are known as tactic factors for leucocytes (neutrophils). When a concentration gradient of such a tactic factor is formed in the hydrogel of the invention and when the hydrogel is contacted with cell groups including leucocytes (e.g., peripheral blood), then the cell of higher taxis to the tactic factor induced by the tactic factor thereof are separated from those of lower taxis to it inside the hydrogel in accordance with the concentration of the tactic factor in the hydrogel. Next, a part of the hydrogel in which the targeted cells exist is cut out and cooled so that the hydrogel is converted into a sol state thereof, and the resulting sol is diluted with physiological saline solution, and thereafter the targeted cell only may be collected through centrifugation.

When a large number of cell having an affinity to fMLP or LPS are desired to be collected from blood, then the tactic factor is dissolved in an aqueous solution of the hydrogel of the invention that is in a sol state at a low temperature, and, for example, the aqueous solution is dropped into a physiological saline solution at a temperature higher than the sol-gel transition temperature of the hydrogel and is thereby gelled in the form of fine drops, and the fine drops of the hydrogel of the invention that contains the tactic factor are collected from the physiological saline solution while kept at a temperature higher than the sol-gel transition temperature thereof, and then they are dispersed in blood. As such, this is stirred while still kept at a temperature higher than the sol-gel transition temperature, then only the cells having an affinity to fMLP or LPS transfer into the hydrogel of the invention that is in the form of fine drops. Kept at a temperature higher than the sol-gel transition temperature thereof, the fine drops of the hydrogel having taken the cell therein are collected through centrifugation, and these are washed. Next, the hydrogel of the invention thus having taken the cell therein are cooled to a temperature lower than the transition temperature thereof so that it is converted into a sol state, then this is diluted with physiological saline solution and repeatedly washed. In that manner, the hydrogel of the invention is removed and the targeted cells are collected. The process is characterized in that, since not only cell but also the fine drops of the hydrogel of the invention containing a tactic factor may freely move in the cell suspension and since the hydrogel of the invention can produce an extremely large surface area per unit volume because of its size and shape, high-frequency contact between cell and the hydrogel of the invention is expected. In addition, various tactic factors may be embedded into the hydrogel of the invention and the condition including time and temperature may be varied, whereby small-sized and multiplex-type functional collection method can be expected.

(Fractionation and collection of high-homing cell for stem cell transplantation)

In transplantation of human hematopoietic stem cell, it is known that hematopoietic stem cell of multi-differentiability are included in CD34-positive cell in hematopoietic cell, and the CD34 positivity of the hematopoietic cell of the donor transplantation is utilized as one criterion for evaluation of hematopoietic stem cell transplantation. Recently, it has been specifically noticed that, among the CD34-positive cells, the cells having strong taxis to kemokines such as stromal cell derived factor-1 (SDF-1), which is a ligand of a kemochine receptor CXCR-4, have a high homing activity for a recipient in the bone marrow transplantation. There is a report saying that, when the number of cells having a high taxis to SDF-1 is larger, then the homing rate of donor cells in the recipient bone marrow is higher. For evaluating the reactivity to SDF-1 of CD34-positive cells and for separating the cells reactive to SDF-1, the hydrogel of the invention containing SDF-1 is utilized. From the hematopoietic cell collected from the marrow fluid, the peripheral blood or the cord blood of a transplantation donor, CD34-positive cells are separated by a magnetic bead method. Then the hydrogel of the invention containing SDF-1 is co-suspended in a suspension of the CD34-positive cells, stirred and cultured for a predetermined period of time, and the CD34-positive cells having reacted to SDF-1 and entered the hydrogel of the invention are separated and collected. The recovery rate indicates the proportion of SDF-1-reactive cells, and is expected to be able to evaluate the homing activity of the donor cells. In addition, the thus-separated cell are directly usable for transplantation as hematopoietic stem cell having a high-homing activity.

(Functional separation and in-vitro fertilization based on the taxis of sperms)

Sperms are cell specialized for fertilization, having flagella and showing taxis of a sophisticated motility function, and the cell is only one type that is released from the individual thereof to play an important role in the life of an organism. The taxis of the sperm enables fertilization. For utilizing only sperms having a high migration capability based on the taxis thereof for fertilization with egg cell, the hydrogel of the invention is utilized. A sperm fluid collected is washed, and the sperms are suspended in a small amount of the hydrogel of the invention that is in a sol state, and this is gelled as such at a temperature higher than the sol-gel transition temperature of the hydrogel. Then, the hydrogel of the invention which is in the form of fine drops and contains the sperms is covered with a suitable amount of the hydrogel of the invention prepared separately having a suitable concentration. The amount and the concentration of the outer coated hydrogel are so controlled that only sperms having a high migration capability may move in the hydrogel and that they can move out through the hydrogel into the external suspension within a predetermined period of time. The drops of the hydrogel of the invention containing sperms are co-suspended in a plastic dish with egg cell in a culture medium, at a temperature higher than the sol-gel transition temperature of the hydrogel. Only the sperms having a high motility function show high taxis in the hydrogel of the invention, and only the sperms functionally selected in the hydrogel of the invention and showing high taxis go out into the culture medium. Accordingly, the egg cell can be fertilized with only the high-taxis sperms.

The invention is described more concretely with reference to the following Examples. However, the scope of the invention is restricted by the claims but not by the following Examples.

EXAMPLES

Examples

Hereinbelow, the present invention will be described in more detail with reference to Examples. However, it should be noted that the present invention is defined by Claims, but is not limited by the following Examples.

Production Example 1

10 g of a polypropylene oxide-polyethylene oxide copolymer (average polymerization degree of propylene oxide/ethylene oxide = about 60/180, Pluronic F-127, mfd. by Asahi Denka K.K.) was dissolved in 30 ml of dry chloroform, and in the co-presence of phosphorus pentaoxide, 0.13g of hexamethylene diisocyanate was added thereto, and the resultant mixture was subjected to reaction under refluxing at the boiling point for six hours. The solvent was distilled off unde reduced pressure, the resultant residue was dissolved in distilled water, and subjected to ultrafiltration by using an ultrafiltration membrane having a molecular cutoff of $3 \times 10^4$ (Amicon PM-30) so as to fractionate the product into a low-molecular weight polymer fraction and a high-molecular weight polymer fraction. The resultant aqueous solution was frozen, to thereby obtain a high-polymerization degree product of F-127 and a low-polymerization degree product of F-127.

When the above high-polymerization degree product of F-127 (TGP-1, a hydrogel-forming polymer according to the present invention) was dissolved in distilled water under ice-cooling in concentration of 8 mass %. When the resultant aqueous solution was gradually warmed, it was found that the viscosity was gradually increased from 21 °C, and was solidified at about 27 °C so as to be converted into a hydrogel state. When the resultant hydrogel was cooled, it was returned to an aqueous solution at 21 °C. Such a conversion was reversibly and repetitively observed. On the other hand, a solution which had been obtained by dissolving the above low-polymerization degree product of F-127 in distilled water under ice-cooling in a concentration of 8 mass %, was not converted into a gel state at all even when it was heated to 60 °C or higher.

Production Example 2

160 mol of ethylene oxide was subjected to an addition reaction with 1 mol of trimethylol propane by cationic polymerization, to thereby obtain polyethylene oxide triol having an average molecular weight of about 7000.

100 g of the thus obtained polyethyleneoxide triol was dissolved in 1000 ml of distilled water, and then 12 g of potassium permanganate was slowly added thereto at room temperature, and the resultant mixture was subjected to an oxidization reaction at this temperature for about one hour. The resultant solid content was removed by filtration, and the product was subjected to extraction with chloroform, and the solvent (chloroform) was distilled off, to thereby obtain 90 g of a polyethylene oxide tricarboxyl derivative.

10 g of the thus obtained polyethylene oxide tricarboxyl derivative, and 10 g of polypropylene oxide diamino derivative (average propylene oxide polymerization degree: about 65, trade name: Jeffamine D-4000, mfd. by Jefferson Chemical Co., U.S.A., cloud point: about 9 °C) were dissolved in 1000 ml of carbon tetrachloride, and then 1.2 g of dicyclohexyl carbodiimide was added thereto, and the resultant mixture was allowed to cause a reaction for 6 hours under refluxing at boiling point. The resultant reaction mixture was cooled and the solid content was removed by filtration, and thereafter the solvent (carbon tetrachloride) therein was distilled off under reduced pressure. Then, the resultant residue was dried under vacuum, to thereby obtain a polymer for coating (TGP-2), a hydrogel-forming polymer according to the present invention comprising plural polypropylene oxide blocks, and polyethylene oxide block combined therewith. This polymer was dissolved in distilled water under cooling with ice so as to provide a concentration of 5 mass %. When the sol-gel transition temperature of the resultant aqueous solution was measured, it was found that the sol-gel transition temperature was about 16 °C.

Production Example 3

96 g of N-isopropyl acrylamide (mfd. by Eastman Kodak Co.), 17 g of N-aclyloxy succinimide (mfd. by Kokusan Kagaku K.K.), and 7 g of n-butyl methacrylate (mfd. by Kanto Kagaku K.K.) were dissolved in 4000 ml of chloroform. After the purging with nitrogen gas, 1.5 g of N,N'-azobisisobutyronitrile was added thereto, and the resultant mixture was subjected to polymerization at 60 °C for 6 hours. The reaction mixture was concentrated, and then was reprecipitated in diethyl ether. The resultant solid content was recovered by filtration, and then was dried under vacuum, to thereby obtain 78 g of poly (N-isopropyl acrylamide-co-N-aclyloxy succinimide-co-n-butyl methacrylate).

Then, an excess of isopropylamine was added to the thus obtained poly(N-isopropyl acrylamide-co-N-aclyloxy succinimide-co-n-butyl methacrylate) to thereby obtain poly(N-isopropyl acrylamide-co-n-butyl methacrylate). The thus obtained poly(N-isopropyl acrylamide-co-n-butyl methacrylate) had a cloud point of about 19 °C in its aqueous solution.

Then, 10 g of the thus obtained poly(N-isopropyl acrylamide-co-N-aclyloxy succinimide-co-n-butyl methacrylate) and 5 g of both terminal-aminated polyethylene oxide (molecular weight = 6000, mfd. by Kawaken Fine Chemical K.K.) were dissolved in 1000 ml of chloroform, and the resultant mixture was allowed to cause a reaction at 50 °C for 3 hours. The reaction mixture was cooled to room temperature, and thereafter 1 g of isopropylamine was added thereto, and was left standing for 1 hour. The reaction mixture was concentrated, and then was precipitated in diethyl ether. The solid content was recovered by filtration, and thereafter was dried under vacuum, to thereby obtain a polymer for coating (TGP-3), a hydrogel-forming polymer according to the present invention comprising plural poly(N-isopropyl acrylamide-co-n-butyl methacrylate) blocks and polyethylene oxide block combined therewith.

This polymer was dissolved in distilled water under cooling with ice so as to provide a concentration of 5 mass %. When the sol-gel transition temperature of the resultant aqueous solution was measured, it was found that the sol-gel transition temperature was about 21 °C.

Production Example 4

(Sterilization method)

2.0g of the above-mentioned polymer (TGP-1) was placed in an EOG (ethylene oxide gas) sterilizing bag (trade name: Hybrid Sterilization bag, mfd. by Hogi Medical Co.), and was filled up with EOG by use of an EOG sterilizing device (trade name: Easy Pack, mfd. Inouchi Seieido Co.) for sterilization and then the bag was left standing at room temperature for twenty-four hours. Further, the bag was left standing at 40 °C for half a day, EOG was removed from the bag and the bag was subjected to aeration. The bag was placed in a vacuum drying chamber (40 °C) and was left standing for half a day, and was sterilized while the bag was sometimes subjected to aeration.
Separately, it was confirmed that the sol-gel transition temperature of the polymer was not changed even after this sterilization operation.

Production Example 5

37 g of N-isopropylacrylamide, 3 g of n-butyl methacrylate, and 28 g of polyethylene oxide monoacrylate (having a molecular weight of 4,000, PME-4000 mfd. by Nihon Yushi K.K. (NOF Corporation)) were dissolved in 340 mL of benzene. Thereafter, 0.8 g of 2,2'-azobisisobutyronitrile was added to the resultant solution, and then was subjected to a reaction at 60°C for 6 hours. 600 mL of chloroform was added to the thus obtained reaction product so as to be dissolved therein, and the resultant solution was dropped into 20 L (liter) of ether so as to be precipitated therein. The resultant precipitate was recovered by filtration, and the precipitate was then subjected to vacuum drying at about 40°C for 24 hours. Thereafter, the resultant product was again dissolved in 6 L of distilled water. The solution was concentrated to a volume of 2 L at 10°C by using a hollow fiber ultrafiltration membrane with a molecular weight cutoff of $10 \times 10^4$ (H1P100-43 mfd. by Amicon),.
The concentrated solution was diluted with 4 L of distilled water, and then, the dilution operation was carried out again. The above dilution and concentration by ultrafiltration were further repeated 5 times, so as to eliminate products having a molecular weight of $10 \times 10^4$ or lower. The product which had not been filtrated by this ultrafiltration (i.e., the product remaining in the inside of the ultrafiltration membrane) was recovered and freeze-dried, so as to obtain 60 g of a hydrogel-forming polymer (TGP-4) according to the present invention having a molecular weight of $10 \times 10^4$ or higher.
1 g of the thus obtained hydrogel-forming polymer (TGP-4) according to the present invention was dissolved in 9 g of distilled water under ice cooling. When the sol-gel transition temperature of the obtained aqueous solution was measured, it was found to be 25°C.

Production Example 6

The hydrogel-forming polymer (TGP-3) according to the present invention which had been obtained in Production Example 3 was dissolved so as to provide a concentration of 10 mass % in distilled water. When the steady flow viscosity η thereof at 37°C was measured, it was found to be $5.8 \times 10^5$ Pa·sec.
On the other hand, agar was dissolved so as to provide a concentration of 2 mass % in distilled water at 90°C, and the mixed solution was converted into a gel state at 10°C for 1 hour. Thereafter, η thereof at 37°C was measured. As a result, the obtained value exceeded the measurement limit ($1 \times 10^7$ Pa·sec) of the apparatus.

Production Example 7

71.0 g of N-isopropylacrylamide and 4.4 g of n-butyl methacrylate were dissolved in 1,117 g of ethanol. To the resultant mixture solution, an aqueous solution which had been obtained by dissolving 22.6 g of polyethylene glycol dimethacrylate (PDE 6000, mfd. by NOF Corporation) in 773 g of water was added. The resultant solution was heated to 70°C under a nitrogen stream. While the resultant solution was maintaining at 70°C under a nitrogen stream, 0.8 mL of N,N,N',N'-tetramethylethylenediamine (TEMED) and 8 mL of 10 % ammonium persulfate (APS) aqueous solution were added to the solution, and then was subjected to a reaction for 30 minutes under stirring. Further, 0.8 mL of TEMED and 8 mL of 10 % APS aqueous solution were added thereto 4 times at 30-minute intervals, and the polymerization reaction was terminated. The reaction mixture was cooled to 10°C or lower, it was diluted with 5 L of cold distilled water with a temperature of 10°C. Thereafter, the solution was concentrated to 2 L at 10°C, by using an ultrafiltration membrane with a molecular weight cutoff of $10 \times 10^4$.
4 L of cold distilled water was added to the concentrated solution for dilution, and the above concentration operation using the ultrafiltration was conducted again. Thereafter, the above dilution and ultrafiltration concentration were repeated 5 times, so as to eliminate products with a molecular weight of $10 \times 10^4$ or lower. The product which had not been filtrated by the above ultrafiltration (product remaining in the ultrafiltration membrane) was recovered and freeze-dried, so as to obtain 72 g of the hydrogel-forming polymer (TGP-5) according to the present invention with a molecular weight of $10 \times 10^4$ or higher.

1 g of the thus obtained hydrogel-forming polymer (TGP-5) according to the present invention was dissolved in 9 g of distilled water under ice cooling. When the sol-gel transition temperature of this aqueous solution was measured, it was found to be 20°C.

Production Example 8

42.0 g of N-isopropylacrylamide and 4.0 g of n-butyl methacrylate were dissolved in 592 g of ethanol. To the resultant mixture solution, an aqueous solution which had been obtained by dissolving 11.5 g of polyethylene glycol dimethacrylate (PDE 6000, mfd. by NOF Corporation) in 65.1 g of water was added. The resultant solution was heated to 70°C under a nitrogen stream. While the resultant solution was maintained at 70°C under a nitrogen stream, 0.4 mL of N,N,N',N'-tetramethylethylenediamine (TEMED) and 4 mL of 10 % ammonium persulfate (APS) aqueous solution were added to the solution, and then, the thus obtained solution was subjected to a reaction for 30 minutes under stirring. Further, 0.4 mL of TEMED and 4 mL of 10 % APS aqueous solution were added thereto 4 times at 30-minute intervals, and the polymerization reaction was terminated. The reaction mixture was cooled to 5°C or lower, it was diluted with 5 L of cold distilled water with a temperature of 5°C. Thereafter, the solution was concentrated to 2 L at 5°C, by using an ultrafiltration membrane with a molecular weight cutoff of $10 \times 10^4$.

4 L of cold distilled water was added to the concentrated solution for dilution, and the above concentration operation using the ultrafiltration was conducted again. Thereafter, the above dilution and ultrafiltration concentration were repeated 5 times, so as to eliminate the product with a molecular weight of $10 \times 10^4$ or lower. The product which had not been filtrated by the above ultrafiltration (product remaining in the ultrafiltration membrane) was recovered and freeze-dried, so as to obtain 40 g of the hydrogel-forming polymer (TGP-6) according to the present invention with a molecular weight of $10 \times 10^4$ or higher.

1 g of the thus obtained hydrogel-forming polymer (TGP-6) according to the present invention was dissolved in 9 g of distilled water under ice cooling. When the sol-gel transition temperature of this aqueous solution was measured, it was found to be 7°C.

Production Example 9

45.5 g of N-isopropylacrylamide and 0.56 g of n-butyl methacrylate were dissolved in 592 g of ethanol. To the resultant mixture solution, an aqueous solution which had been obtained by dissolving 11.5 g of polyethylene glycol dimethacrylate (PDE 6000, mfd. by NOF Corporation) in 65.1 g of water was added. The resultant solution was heated to 70°C under a nitrogen stream. While the solution was maintained at 70°C under a nitrogen stream, 0.4 mL of N,N,N',N'-tetramethylethylenediamine (TEMED) and 4 mL of 10 % ammonium persulfate (APS) aqueous solution were added to the solution, and then was subjected to a reaction for 30 minutes under stirring. Further, 0.4 mL of TEMED and 4 mL of 10 % APS aqueous solution were added thereto 4 times at 30-minute intervals, and the polymerization reaction was terminated. The reaction mixture was cooled to 10°C or lower, it was diluted with 5 L of cold distilled water with a temperature of 10°C. Thereafter, the solution was concentrated to 2 L at 10°C, by using an ultrafiltration membrane with a molecular weight cutoff of $10 \times 10^4$.

4 L of cold distilled water was added to the concentrated solution for dilution, and the above concentration operation using the ultrafiltration was conducted again. Thereafter, the above dilution and ultrafiltration concentration were repeated 5 times, so as to eliminate the product with a molecular weight of $10 \times 10^4$ or lower. The product which had not been filtrated by the above ultrafiltration (product remaining in the ultrafiltration membrane) was recovered and freeze-dried, so as to obtain 22 g of the hydrogel-forming polymer (TGP-7) according to the present invention with a molecular weight of $10 \times 10^4$ or higher.

1 g of the thus obtained hydrogel-forming polymer (TGP-7) according to the present invention was dissolved in 9 g of distilled water under ice cooling. When the sol-gel transition temperature of this aqueous solution was measured, it was found to be 37°C.

Example 1

(Migration capability of neutrophils)

Soft agar mediums (prepared by dissolving Nacalai Tesque's soft agar powder in a D'MEM (Dulbecco's Modification Eagle's Medium, by GIBCO, containing 10 % FCS (Fetal Calf Serum)) having 0.6% of concentration and each containing 0 M, $10^{-6}$ M, $10^{-7}$ M or $10^{-8}$ M of fMLP (N-formyl-methionyl-leucyl-phenylalanine, molecular weight 437.6, chemotactic peptide, produced by Sigma) were prepared at 42°C. Each medium was put into a polystyrene dish (mfd. by SUMILON) having a diameter of 35 mm in an amount of 1 ml (depth of medium, about 1 mm) each, and gelled therein at room temperature. The hydrogel-forming polymer (TGP-5) of the invention obtained in Production Example 7 was sterilized with EOG in the same manner as in Production Example 4. One g of the polymer was dissolved in 9 g of D'MEM medium at 4°C, and this was put on the above-mentioned soft agar gel in an amount of 0.5 ml (depth of the polymer solution, about 0.5 mm) each, and gelled at room temperature. The sol-gel transition temperature of the hydrogel was 18°C. On the hydrogel layer of the invention, a tissue culture insert (mfd. by NUNC) with a filter having a pore size of 8.0 μm was set, and 0.5 ml of a cell suspension containing $10^7$ human peripheral blood leucocytes was put into the insert and set in an incubator at 37°C. This was statically set therein at 37°C for 3 hours, and then the dish was taken out of the incubator. The dish was cooled on ice, the tissue culture insert was removed, and the hydrogel of the invention was converted into a sol state. The hydrogel of the invention that was liquid with cooling with ice was collected, diluted with physiological saline and centrifuged (3000 rpm, 5 minutes). The precipitated cell were re-suspended in 20 μl of physiological saline, and the number of the cell was counted to thereby determine the number of the neutrophils having moved into the hydrogel of the invention. After the hydrogel of the invention was removed, the number of the cell having moved into the soft agar was microscopically observed, and the result is shown in Table 1.

(Tactic factor concentration and number of cell having moved in hydrogel)

Table 1 -

| Tactic Factor Concentration and Number of Cell Having Moved in Hydrogel | | | | |
|---|---|---|---|---|
| fMLP Concentration in Soft Agar | 0 M | $10^{-8}$ M | $10^{-7}$ M | $10^{-6}$ M |
| Number of Cells in Hydrogel of the Invention | 3,240 | 4,740 | 17,600 | 300 |
| Number of Cells in Soft Agar | no | a few | many | numerous |

The result in the above Table 1 shows that, when the concentration of the tactic factor fMLP in the gel is higher, then migration capability of the fMLP-reactive cells is higher. In addition, it also shows that the cells move in the direction of a higher fMLP concentration.

Example 2

(Selective fractionation and collection of neutrophils)

The hydrogel-forming polymer (TGP-5) of the invention obtained in Production Example 7 was sterilized with EOG in the same manner as in Production Example 4. One g of the polymer was dissolved in 9 g of D'MEM (Dulbecco's Modification Eagle's Medium, by GIBCO, containing 10 % fetal calf serum) with cooling with ice. The sol-gel transition temperature of the aqueous solution was measured, and it was 18°C.
fMLP was dissolved in the D'MEM with the hydrogel-forming polymer (TGP-5) therein, at 4°C (concentration: $10^{-6}$ M). The fMLP-containing hydrogel-forming polymer (TGP-5) D'MEM was put into a 1-ml syringe equipped with a 23G needle, and cooled to 4°C. One ml of the aqueous solution at 4°C was extruded out into 10 ml of phosphate-buffered saline (PBS) at 37°C in a disposable centrifugal tube (by Falcon, 14 ml), thereby forming a string-like hydrogel. While kept at 37°C, PBS was removed through decantation with the string-like hydrogel left in the tube, and in place of it, 10 ml of heparinized human whole blood was added to the string-like hydrogel in the centrifugal tube and gently rotated and stirred at 37°C for 4 hours. Still kept at 37°C, the heparinized human whole blood was removed, and the string-like hydrogel was washed with PBS warmed at 37°C. After washed, the string-like hydrogel was cooled to 4°C and formed into a sol state. This was diluted with PBS and centrifugally washed, and a Wright's Giemsa stained specimen of the cells which entered the gel was prepared and observed with a microscope. As a result, only cell which have reacted with fMLP and which have moved into the gel were observed. Neutrophils and monocytes were superior in the cell fraction, and few of cell non-reactive to fMLP such as erythrocytes and thrombocytes were found.

Comparative Example

The same experiment as in Example 2 was carried out, in which, however, fMLP was not contained in the hydrogel. The cell taken into the hydrogel in this Comparative Example was observed, and few of erythrocytes, thrombocytes and leucocytes were found.

INDUSTRIAL APPLICABILITY

As described hereinabove, the present invention provides a hydrogel capable of conducting separation (fractionation, differential separation, fractional collection) of cell/organism having a variety of taxes.

**Claims**

1. A hydrogel for separating cell/organism, which is capable of selectively moving a cell/organism in accordance with a difference in the concentration of a physiologically active substance.

2. A hydrogel for separating cell/organism according to claim 1, wherein the selective movement is that between the gel and the external environment surrounding the gel.

3. A hydrogel for separating cell/organism according to claim 1, wherein the selective movement is that in the inside of the gel.

4. A hydrogel for separating cell/organism according to any of claims 1 to 3, wherein the hydrogel shows a thermo-reversible sol-gel transition such that it assumes a sol state at a lower temperature and assumes a gel state at a higher temperature, and the gel is substantially insoluble in water at a temperature higher than the sol-gel transition temperature thereof.

5. A hydrogel for separating cell/organism according to claim 4, wherein the hydrogel has a sol-gel transition temperature higher than 0°C but not higher than 45°C.

6. A hydrogel for separating cell/organism according to any of claims 1 to 5, wherein the hydrogel contains a physiologically active substance and the physiologically active substance has a concentration difference between the inside and outside of the hydrogel.

7. A hydrogel for separating cell/organism according to any of claims 1 to 5, wherein the hydrogel does not substantially contain a physiologically active substance and the physiologically active substance has a concentration difference between the inside and outside of the hydrogel.

8. A hydrogel for separating cell/organism according to any of claims 1 to 7, wherein a concentration gradient of the physiologically active substance is provided in the inside of the hydrogel.

9. A hydrogel for separating cell/organism according to any of claims 1 to 7, wherein a physical property of field has a gradient, and the cell/organism is separated in accordance with a difference in taxis based on the gradient of the physical property.

10. A hydrogel for separating cell/organism according to any of claims 1 to 9, wherein the hydrogel comprises water, and a hydrogel-forming polymer comprising a plurality of blocks having a cloud point and a hydrophilic block combined therewith.

11. A method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,
contacting an aqueous solution containing a physiologically active substance with one face of the gel-forming composition in a gel state, and contacting a suspension of cell/organism with the other face of the composition in a gel state, at a temperature higher than the sol-gel transition temperature of the gel-forming composition, transferring the cell/organism from the suspension into the composition in a gel state due to the chemotaxis induced by a concentration gradient, while providing the concentration gradient of the physiologically active substance in the inside of the composition in a gel state,
separating at least a portion of the composition in a gel state into which the cell/organism has been transferred, from the other portion of the composition, and

cooling the separated portion of the composition in a gel state to a temperature lower than the sol-gel transition temperature of the composition so as to convert the composition into a sol state, to thereby collect the cell/organism from the composition in a sol state.

**12.** A method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,
adding a cell/organism to the gel-forming composition in a sol state at a temperature lower than the sol-gel transition temperature of the composition, to thereby suspend the cell/organism in the composition;
converting the sol-state composition into a gel state at a temperature higher than the sol-gel transition temperature, to thereby provide a composition in a gel state containing the cell/organism substantially uniformly dispersed therein,
contacting the composition in a gel state with an aqueous solution containing a physiologically active substance, at a temperature higher than the sol-gel transition temperature,
re-arranging the cell/organism in the composition in a gel state due to a difference in chemotaxis in accordance with a concentration gradient of the physiologically active substance, while transferring the physiologically active substance into the composition in a gel state so as to provide the concentration gradient of the physiologically active substance in the composition,
separating at least a portion of the composition in a gel state containing the cell/organism which has been re-arranged therein, from the other portion of the composition, and
cooling the separated portion of the composition in a gel state to a temperature lower than the sol-gel transition temperature of the composition so as to convert the composition into a sol state, to thereby collect the cell/organism from the composition in a sol state.

**13.** A method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,
cooling the gel-forming composition to a temperature lower than the sol-gel transition temperature, to thereby convert the composition into a sol state, and substantially uniformly mixing a physiologically active substance in the composition in a sol state,
converting the sol-state composition into a gel state having a predetermined shape, at a temperature higher than the sol-gel transition temperature,
contacting the composition in a gel state with a suspension of cell/organism at a temperature higher than the sol-gel transition temperature, to thereby transfer the cell/organism from the suspension into the composition in a gel state,
separating the composition in a gel state containing the cell/organism having transferred therein, from the suspension of the cell/organism, and
cooling the separated gel-state composition to a temperature lower than the sol-gel transition temperature so as to convert the composition into a sol state, to thereby collect the cell/organism from the composition in a sol state into which the cell/organism has transferred.

**14.** A method of separating cell/organism according to any of claims 11 to 13, wherein the hydrogel-forming composition has a sol-gel transition temperature higher than 0°C but not higher than 45°C.

**15.** A method of separating cell/organism according to any of claims 11 to 14, wherein a plurality of portions of the composition each containing cell/organism which is different in the tactic capability or the moving distance, are separated from the gel-state composition into which the cell/organism has transferred, or in which the cell/organism has been re-arranged, and the cell/organism is collected from the plural portions of the gel-state composition.

**16.** A method of separating cell/organism according to claim 13, wherein the predetermined shape of the hydrogel is such that it provides a ratio of surface area (S)/volume (V) of at least 10 ($cm^{-1}$).

**17.** A method of separating cell/organism according to claim 16, wherein the predetermined shape of the hydrogel is any of sphere, string, fiber, flake, plates, film or indeterminate shape.

**18.** A method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,

positioning the gel-forming composition in a gel state in a field in which a physical property is continuously changed, to thereby provide a gradient of the physical property in the inside of the hydrogel,

contacting the gel-state composition with a suspension of cell/organism, and transferring the cell/organism in the suspension into the gel-state composition due to taxis induced by the gradient of the field property,

separating at least a portion of the gel-state composition containing the cell/organism having transferred therein, from the other portion of the composition, and

cooling the separated portion of the gel-state composition to a temperature lower than the sol-gel transition temperature of the composition so as to convert the composition into a sol state, to thereby collecting the cell/organism from sol-state the composition.

**19.** A method of separating cell/organism, comprising:

providing a gel-forming composition comprising at least water, and a hydrogel-forming polymer; the gel-forming composition being capable of reversibly assuming a sol state at a temperature lower than the sol-gel transition temperature, and being capable of assuming a substantially water-insoluble gel state at a temperature higher than the sol-gel transition temperature,

converting the gel-forming composition into a sol state at a temperature lower than the sol-gel transition temperature, and adding cell/organism to the sol, to thereby form a composition containing the cell/organism suspended therein,

converting the composition containing the cell/organism suspended therein, into a gel state at a temperature higher than the sol-gel transition temperature, to thereby form a composition in a gel state in which the cell/organism is substantially uniformly dispersed,

positioning the gel-forming composition in a gel state in a field in which a physical property is continuously changed, to thereby provide a gradient of the physical property in the inside of the gel,

re-arranging the cell/organism in the gel-state composition, which has been substantially uniformly distributed therein, in accordance with the gradient of the physical property and with a difference of the taxis of the cell/organism to the physical property,

separating at least a portion of the gel-state composition containing the cell/organism re-arranged therein, from the other portion of the composition, and

cooling the separated portion of the gel-state composition to a temperature lower than the sol-gel transition temperature of the composition so as to convert the composition into a sol state, to thereby collect the cell/organism from sol-state the composition.

**20.** A method of separating cell/organism according to claim 18 or 19, wherein the physical property is at least one selected from electric field intensity, magnetic field intensity, light intensity, temperature, and viscosity.

**21.** A method of separating cell/organism according to any of claims 18 to 20, a plurality of portions of the composition each containing cell/organism which is different in the tactic capability or the moving distance, are separated from the gel-state composition into which the cell/organism has transferred, or in which the cell/organism has been re-arranged, and the cell/organism is collected from the plural portions of the gel-state composition.

**22.** A method of separating cell/organism according to any of claims 18 to 21, wherein the hydrogel-forming composition has a sol-gel transition temperature higher than $0°C$ but not higher than $45°C$.

# Fig.1

CHEMOTACTIC FACTOR:
HIGH-CONCENTRATION SIDE

POSITIVE
CHEMOTAXIS

NEGATIVE
CHEMOTAXIS

CHEMOTACTIC FACTOR:
LOW-CONCENTRATION SIDE

CHEMOTAXIS-BASED
FRACTIONATION

CUTTING-OUT OF GEL

COOLING AND
DISSOLUTION

COLLECTION
OF CELLS

# Fig.2

CHEMOTACTIC FACTOR-
CONTAINING HYDROGEL
OF THE INVENTION

SUSPENSION OF CELLS/ORGANISMS

COOLING AND
DISSOLUTION

CELL SUSPENSION IS
CONTACTED WITH
HYDROGEL OF THE
INVENTION CONTAINING
CHEMOTACTIC FACTOR

CELLS HAVING AFFINITY
TO CHEMOTACTIC FACTOR
ARE SELECTIVELY TAKEN
INTO HYDROGEL OF THE
INVENTION

HYDROGEL OF THE INVENTION CONTAINING
FRACTIONATED CELLS IS SEPARATED FROM
CELL SUSPENSION, GEL IS CONVERTED INTO
A SOL PHASE BY COOLING, AND FRACTIONATED
CELLS ARE COLLECTED

EP 1 595 944 A1

# EP 1 595 944 A1

<table>
<tr><td colspan="2" style="text-align:center"><b>INTERNATIONAL SEARCH REPORT</b></td><td>International application No.<br>PCT/JP2004/001856</td></tr>
</table>

**A. CLASSIFICATION OF SUBJECT MATTER**
Int.Cl$^7$ C12N5/00, C08L101/14

According to International Patent Classification (IPC) or to both national classification and IPC

**B. FIELDS SEARCHED**

Minimum documentation searched (classification system followed by classification symbols)
Int.Cl$^7$ C12N5/00, C08L101/14, C12M1/00-3/10

Documentation searched other than minimum documentation to the extent that such documents are included in the fields searched

Electronic data base consulted during the international search (name of data base and, where practicable, search terms used)
WPIDS, JSTplus

**C. DOCUMENTS CONSIDERED TO BE RELEVANT**

| Category* | Citation of document, with indication, where appropriate, of the relevant passages | Relevant to claim No. |
|---|---|---|
| X/<br>Y | US 5733736 A  (Springfield Collage),<br>31 March, 1998 (31.03.98),<br>& WO 98/27431 A      & AU 4229997 A<br>& CA 2275623 A      & EP 956505 A<br>& JP 2001-527640 A | 1-3,8/<br>4-7,9-22 |
| Y | JP 11-276156 A  (Mitsubishi Electric Corp.),<br>12 October, 1999 (12.10.99),<br>(Family: none) | 1-22 |
| Y | WO 00/07007 A  (Biometric Imaging Inc.),<br>10 February, 2000 (10.02.00),<br>& EP 1101106 A          & JP 2002-521690 A | 1-22 |

[X] Further documents are listed in the continuation of Box C.     [ ] See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>24 March, 2004 (24.03.04) | Date of mailing of the international search report<br>13 April, 2004 (13.04.04) |
|---|---|
| Name and mailing address of the ISA/<br>Japanese Patent Office | Authorized officer |
| Facsimile No. | Telephone No. |

Form PCT/ISA/210 (second sheet) (January 2004)

## INTERNATIONAL SEARCH REPORT

| | International application No. |
| --- | --- |
| | PCT/JP2004/001856 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | US 6391654 A  (Genosis Ltd.),<br>21 May, 2002 (21.05.02),<br>& WO 00/09648 A         & AU 5431999 A<br>& BR 9913039 A          & CA 2333900 A<br>& DE 6990383 A          & EP 1104454 A | 1-22 |
| Y | JP 2002-159287 A  (Effector Cell Institute),<br>04 June, 2002 (04.06.02),<br>(Family: none) | 1-22 |
| Y | JP 8-23956 A  (Matsushita Electric Industrial Co.,<br>Ltd.),<br>30 January, 1996 (30.01.96),<br>(Family: none) | 1-22 |
| Y | JP 2001-17156 A  (Matsushita Electric Industrial<br>Co., Ltd.),<br>23 January, 2001 (23.01.01),<br>(Family: none) | 1-22 |
| Y | GB 2314035 A  (Mini Agriculture & Fisheries),<br>17 December, 1997 (17.12.97),<br>& WO 96/25998 A         & AU 4728696 A<br>& CA 2213458 A          & EP 1810900 A<br>& JP 11-501210 A | 1-22 |
| Y | JP 2000-262865 A  (Matsushita Electric Industrial<br>Co., Ltd.),<br>26 September, 2000 (26.09.00),<br>(Family: none) | 1-22 |
| Y | EP 214340 A  (Biocontrol Systems Inc.),<br>18 March, 1987 (18.03.87),<br>& CA 1271707 A          & DE 3585689 A<br>& JP 62-61598 A | 1-22 |

Form PCT/ISA/210 (continuation of second sheet) (January 2004)